Europäisches Patentamt

European Patent Office

Office européen des brevets

(11) Publication number: **0122249** B1

# EUROPEAN PATENT SPECIFICATION

(45) Date of publication of the patent specification:
21.05.86

(21) Application number: **84870046.4**

(22) Date of filing: **29.03.84**

(51) Int. Cl.⁴: **C 07 C 55/14,** C 07 C 51/47,
C 07 C 51/31, C 07 C 51/487,
B 01 J 23/92

(54) Recovery of vanadium and copper from adipic acid production.

(30) Priority: **01.04.83 US 481220**

(43) Date of publication of application:
**17.10.84 Bulletin 84/42**

(45) Publication of the grant of the patent:
**21.05.86 Bulletin 86/21**

(84) Designated Contracting States:
**BE DE FR GB**

(56) References cited:
**CH - A - 301 794**
**DE - A - 1 290 928**
**GB - A - 1 526 927**

(73) Proprietor: **MONSANTO COMPANY, Patent Department 800 North Lindbergh Boulevard, St. Louis, Missouri 63166 (US)**

(72) Inventor: **Hsu, Charles Kou-Chao, 4860 Balmoral Drive, Pensacola Florida 32504 (US)**
Inventor: **Laird, Donald Edward, 2646 Broome Circle, Pensacola Florida 32504 (US)**

(74) Representative: **McLean, Peter et al, Monsanto Europe S.A. Patent Department Avenue de Tervuren 270-272 Letter Box No 1, B-1150 Brussels (BE)**

## Description

## BACKGROUND OF THE INVENTION

### A. Field of the Invention

The production of adipic acid bye the liquid phase nitric acid oxidation of cyclohexanol and/or cyclohexanone in the presence of a vanadium catalyst usually results in a purge waste stream containing valuable vanadium ions. The purge waste stream is generally derived from the mother liquor of one or more crystallizations involved in the recovery of the adipic acid and the necessity for purging arises because of the buildup of other dibasic acids such as succinic acid and glutaric acid. In view of the value of such catalyst metals, recovery of both from the purge waste stream is necessary, if the process is to operate economically.

### B. The Prior Art.

The process for the nitric acid oxidation of cyclohexanol and/or cyclohexanone in the presence of copper and vanadium catalysts is well known. The feed mixture utilized in most industrial processes is a mixture of cyclohexanone and cyclohexanol derived from the air oxidation of cyclohexane although some processes utilize only cyclohexanol or only cyclohexanone as a feed to the nitric acid oxidation. The cyclohexanol and/or cyclohexanone is generally mixed with from 5 to 40 times its weight of an aqueous nitric acid solution, the nitric acid solution being generally of a concentration of 35 to 65% by weight and there is also added the metal catalyst. Usually copper and vanadium are added as $V_2O_5$ or ammonium metavanadate and copper turnings. The total amount of catalyst is usually about 0.05 to 1.0% by weight of the reaction mixture. The liquid phase nitric acid oxidation is generally conducted at temperatures within the range of about 55° to 100°C, and pressures within the range of about 1 to 5 atmospheres absolute.

There is produced in the nitric acid oxidation a liquid reaction product comprising the adipic acid as well as succinic acid, glutaric acid, nitric acid, water and the copper and vanadium catalyst values. There is also produced in the nitric acid oxidation an off-gas containing nitrogen oxides such as NO, $N_2O$, $NO_2$ and $N_2O_4$ as well as other gases such as carbon dioxide and nitrogen. Adipic acid crystals are recovered from the liquid reaction product by crystallization techniques, resulting in a mother liquor which comprises an aqueous nitric acid solution containing copper and vanadium values as well as dibasic carboxylic acids (mainly glutaric and succinic acids although some unrecovered adipic acid may also be present). A portion of this mother liquor may be, and generally is, recycled to the nitric acid oxidation reactor although a portion must be removed or purged to prevent buildup of the succinic and glutaric acid impurities, the portion removed or purged being the above-mentioned purge waste stream. The recovery of the adipic acid by crystallization is well known and such may be accomplished by one or more crystallization stages and may include effecting a removal of some nitric acid and water and re-dilution between crystallization steps.

Various methods have been developed for recovery of the copper and vanadium values from these purge waste streams as may be seen from U.S. Patent Nos. 3,106,450; 3,186,952; 3,463,740; and 3,554,592. Also see British Patent Specifications 980762 and 936403. The most popular of the methods for the recovery of the catalyst metals from the purge waste streams is by passing these streams through an ion exchange treatment zone whereby the metals are bound to a resin exchanger and then the metal is recovered by passing nitric acid over the exchanger. The stream resulting from the regeneration of the exchanger consists essentially of a nitric acid solution with the catalyst metals therein and, therefore, can be recycled to the nitric acid oxidation zone. While such a recovery process, as well as the various known modifications thereof, are fairly efficient in recovering the copper values from the purge waste streams, they do not provide as efficient recovery of the vanadium values as desired.

It has been proposed in U.S. 3,965,164 to remove $HNO_3$ and water from the adipic mother liquor and then treat the liquor with the adipic byproduct off-gas containing nitrogen oxides, thereby improving recovery of vanadium values. Removal of $HNO_3$ and water, however, has proven difficult and expensive.

Any treatment method for improving ion exchange recovery of vanadium which would not require removal of $HNO_3$ and/or water would be a meritorious advance in the art and is an object of this invention.

## SUMMARY OF THE INVENTION

The invention is an improvement in a process for the production of adipic acid wherein (a) cyclohexanol and/or cyclohexanone are oxidized by nitric acid oxidation at temperatures within the range of 55° to 100°C utilizing a vanadium catalyst and optionalls also a copper catalyst to obtain a liquid reaction product comprising adipic acid and also produce a by-product off-gas containing nitrogen oxides and wherein (b) said reaction product is subjected to crystallization to isolate therefrom an adipic acid product and a mother liquor comprising an aqueous nitric acid solution containing therein dibasic carboxylic acids and vanadium values; which improvement comprises treating at least a protion of the mother liquor to recover vanadium values therefrom by (c) introducing $SO_2$ into the adipic mother liquor feed (AMLF) (ion-exchange feed) so as to improve recovery of vanadium values and, thereafter, (d) recovering vanadium values from the vanadyl-containing mother liquor by passing it through a bed of cation exchange resin so as to accumulate vanadium ions thereon. If copper is also included as a catalyst it may also be recovered by the same cation exchange.

## DETAILED DESCRIPTION OF THE INVENTION

The make-up of the ion exchange feed is well known in the art and will depend on crystallization and evaporation procedures. A typical ion exchange feed contains 1% to 2.5% nitric acid, 20 to 27% alkyl dicarboxylic acids (e.g., 14% glutaric, 6% succinic, 4% adipic) 8000-11,000 ppm copper and 600-2500 ppm vanadium ($V_2O_5$) and the rest is water. Vanadium is introduced in the form of an oxide such as $V_2O_5$, an acid such as vanadic acid, a salt such as $NH_4VO_3$, or in metallic form. If used, copper may be introduced in metallic form. Residual NOx in the feed is preferably removed or converted to nitric acid by air sparging (into the feed tank) before introducing $SO_2$.

While the precise mechanism by which the $SO_2$ increases vanadium recovery is not to be considered a part of or a limitation upon this invention, it is theorized that the $SO_2$ reduces pervanadyl ions to vanadyl ions which have a greater affinity for the ion exchange resin.

Any method of introducing $SO_2$ into the mother liquor is within the scope of this invention.

The apparatus used for contacting a liquid and a gas stream continuously may be a tower filled with irregular solid packing material, an empty tower into which the liquid is sprayed, or a tower containing a number of bubble-cap or sieve plates. Ordinarily, the gas and liquid streams flow countercurrently through the equipment in order to obtain the greatest rate of absorption. The gas absorption operation may also be carried out in one or more spray columns, wetted-wall columns, stirred vessels, or other mechanically aided devices, as well as by simply bubbling the $SO_2$ gas through a container of the liquor.

An open or closed system may be employed but a closed system is preferred. In situ introduction may be achieved by adding chemicals such as metal sulfites or bisulfites to the adipic mother liquor which in turn generate $SO_2$ by internal reaction.

Whatever the means of introduction, the resulting mole ratio of dissolved $SO_2$ to vanadium ion in the ion exchange feed should be about 1-5, preferably 1-3. Below about 1 mole, the effect will be diminished. Above about 5 moles there will be diminishing improvement. The presence of other oxidizing agents such as NOx or ferric ions will lessen diminishing improvement at lower levels.

If only a portion of the ion exchange feed is employed to introduce the $SO_2$, it must, of course, be well mixed with the remainder of the feed. For example, it may be convenient to saturate a stream then mix the saturated stream with the remainder of the feed in a mixing tank.

At a temperature of 60°C, for example, a good mix can be obtained in 10 minutes to 1 hour, either in a closed or open vessel.

A indicated above, the last step of the process of this invention is to ion exchange the solution resulting from mixing sulfurous solution with the ion exchange feed. Any of the hydrogen-form cation exchange resins disclosed in the prior art for recovering metals such as copper and vanadium can be employed. Resins which can be used in alternating cycles of metal absorption followed by mineral acid elution as taught in the prior art can also be employed in the present process. "Amberlite IB-200" manufactured by Rohm and Haas company has been used successfully. Many other similar resins, such as "Dowex 50WX8", "Dowex 50X16" and "Dow SA 1101.1", all manufactured by Dow Chemical Company, can also be used.

## EXAMPLE 1

### (Comparative example)

The above described ion exchange feed was not treated with sulfur dioxide. Ion exchange feed (1500 ml) was heated to 50°C and ion exchanged according to the following conditions and procedures. The catalyst recoveries were shown to be 99% for copper and 55% for vanadium. Ion exchange conditions were as shown in Table I.

## TABLE 1

### Ion-Exchange Conditions

| | |
|---|---|
| Catalyst (ft³) | 0.0353 |
| (liter) | 1.0 |
| Bed Diameter (cm) | 5.08 |
| Bed Height (cm) | 49.28 |
| Feed Cycle (lb) | 2.54 |
| (g) | 1 154.0 |
| Feed (Catalyst, g/l) | |
| Feed Rate | — |
| Average Feed Rate (lb/hr) | — |
| (cc/min) | 113 |
| Feed Flux (g/cm²/min | 6.14 |
| Residence Time (min) | 8 |

### Ion Exchange Operating Procedure

1. Pass 1,000 ml water up through column in order to fluff resin bed. Discard effluent. Avoid carryover of resin.

2. Pass 1,150 grams AMLF down through column at 115 cc/min rate. Discard first 250 ml effluent. Collect rest in AML collection vessel. (Assure AMLF temperature is 45°C).

3. Pass 1,600 ml water down through column at 280 cc/min. Collect effluent in AML collection vessel.

4. Pass 1,500 ml water up through column. Avoid carryover of resin (if no precipitate present in feed, disregard this step). Collect effluent "special collection vessel".

5. Pass 1,825 ml of 30% $HNO_3$ down through column at 280 cc/min. Collect effluent in regenerant collection vessel.

6. Pass 1,000 ml $H_2O$ down through column at 280 cc/min. Collect effluent in regenerant collection vessel.

7. Weigh AML, regenerant, and "special collection" cuts.

8. Submit samples for V, Cu and $HNO_3$ analyses.

## EXAMPLE 2

The same ion exchange feed (300 ml) was sparged with $SO_2$ gas at 50° for 18 minutes to prepare a $SO_2$-saturated solution (the saturation point can be determined analytically). 45 ml of this solution was then added in one portion to a stirred ion exchange feed solution; the resultant solution was stirred for another 30 minutes at 50°C and ion-exchanged according to the normal procedure (see procedure above). The copper and vanadium catalyst recoveries were 99% and 98% respectively.

## EXAMPLE 3

Deionized water (300 ml) was saturated with $SO_2$ at room temperature (25°C). 30 ml of this solution was then added in one portion to a stirred ion exchange feed of the same composition at room temperature. The solution was heated to 50°C, stirred for an additional 30 minutes and passed through the ion exchange column. The copper and vanadium catalyst recoveries were 99% and 98%, respectively.

## EXAMPLE 4

Ion exchange feed (1500 ml) was heated to 50°C. Sodium sulfite (2.6g) was added to the solution; the solution was stirred at 50°C for 20 minutes and ion exchanged. Vanadium recovery was 89% and copper recovery was 99%.

## Claims

1. A process for the production of adipic acid wherein:

(1) Cyclohexanol and/or cyclohexanone are oxidized by nitric acid in the presence of a vanadium catalyst to obtain a liquid reaction product comprising adipic acid,

(2) said reaction product is subjected to crystallization to isolate adipic acid from a mother liquor comprising nitric acid, succinic acid, glutaric acid, residual adipic acid, vanadium, and

(3) vanadium is recovered from the mother liquor by cation exchange,

characterised by the improvement comprising introducing an effective amount of sulfur dioxide into the mother liquor prior cation exchange recovery, so as to increase cation exchange.

2. A process of Claim 1, wherein copper is also included as a catalyst and recovered by the cation exchange.

3. A process of either Claim 1 or Claim 2 wherein the vanadium catalyst is a member of the group consisting of metallic vanadium, an acid of vanadium, an oxide of vanadium and a salt of vanadium.

4. A process of Claim 3, wherein the vanadium catalyst is $V_2O_5$.

5. A process of any of Claims 1 to 5, wherein during ion exchange the $SO_2$ is formed in situ in the mother liquor by the addition of a member of the group consisting of metal sulfites and metal bi-sulfites.

6. A process of any of Claims 1 to 5 wherein during ion exchange the $SO_2$ is present at a mole ratio to vanadium of from 1:1 to 5:1.

7. A process of Claim 6, wherein the mole ratio is from 1:1 to 3:1

8. A process of any of Claims 1 to 7 wherein at least a portion of the process, including the ion exchange, is in a closed system and $SO_2$ is introduced into this portion by sparging.

9. A process of any Claims 1 to 7, wherein the process is conducted in an open system and the $SO_2$ is injected with a conventional water scrubbing unit.

10. A process of any of Claims 1 to 5, wherein $SO_2$ is added to a portion of the ion exchange feed in an amount such as to provide a saturated solution which is in turn added to the ion exchange feed so as to provide a mole ratio of dissolved $SO_2$ to vanadium of from 1:1 to 5:1.

11. A process of Claim 10, wherein the mole ratio of dissolved $SO_2$ to vanadium is from 1:1 to 3:1.

## Patentansprüche

1. Verfahren zur Herstellung von Adipinsäure durch

(1) Oxidation von Cyclohexanol und/oder Cyclohexanon mit Salpetersäure in Gegenwart eines Vanadiumkatalysators unter Bildung eines Adipinsäure enthaltenden flüssigen Reaktionsprodukts,

(2) Kristallisieren des Reaktionsprodukts zur Isolierung der Adipinsäure aus einer Salpetersäure, Bernsteinsäure, Glutarsäure, restliche Adipinsiure und Vanadium enthaltenden Mutterlauge und

(3) Rückgewinnen des Vanadiums aus der Mutterlauge durch Kationenaustausch,

dadurch gekennzeichnet, daß vor der Kationenaustauschrückgewinnung eine wirksame Menge Schwefeldioxid in die Mutterlauge eingeführt wird, um den Kationenaustausch zu steigern.

2. Verfahrennach Anspruch 1, dadurch gekennzeichnet, daß auch Kupfer als Katalysator verwendet und durch den Kationenaustausch zurückgewonnen wird.

3. Verfahren nach Anspruch 1 oder 2, dadurch gekennzeichnet, daß der Vanadiumkatalysator aus der Gruppe ausgewählt ist, die metallisches Vanadium, Vanadiumsäuren, Vanadiumoxide und Vanadiumsalze umfaßt.

4. Verfahren nach Anspruch 3, dadurch gekennzeichnet, daß als Vanadiumkatalysator $V_2O_5$ eingesetzt wird.

5. Verfahren nach einem der Ansprüche 1 bis 5, dadurch gekennzeichnet, daß während des Ionenaustausches durch Zugabe eines Vertreters der Metallsulfite und Metallbisulfite umfassenden Gruppe das $SO_2$ in situ in der Mutterlauge gebildet wird.

6. Verfahren nach einem der Ansprüche 1 bis 5, dadurch gekennzeichnet, daß das $SO_2$ während des Ionenaustausches in einem Molverhältnis in bezug auf Vanadium von 1: 1 bis 5: 1 vorhanden ist.

7. Verfahren nach Anspruch 6, dadurch gekennzeichnet, daß das Molverhältnis sich von 1: 1 bis 3: 1 erstreckt.

8. Verfahren nach einem der Ansprüche 1 bis 7, dadurch gekennzeichnet, daß mindestens ein Abschnitt des Verfahrens, einschließlich des Ionenaustausches, in einem geschlossenen System durchgeführt wird und $SO_2$ durch Einblasen in diesen Bereich eingeführt wird.

9. Verfahren nach einem der Ansprüche 1 bis 7, dadurch gekennzeichnet, daß das Verfahren in einem offenen System durchgeführt wird und $SO_2$ mit Hilfe einer üblichen Wasserwascheinrichtung eingeführt wird.

10. Verfahren nach einem der Ansprüche 1 bis 5, dadurch gekennzeichnet, daß $SO_2$ zu einem Teil der Ionenaustauschbeschickung in einer solchen Menge zugesetzt wird, daß sich eine gesättigte Lösung ergibt, die ihrerseits zu der Ionenaustauschbeschickung zugesetzt wird unter Erzeugung eines Molverhältnis von gelöstem $SO_2$ zu Vanadium von 1: 1 bis 5: 1.

11. Verfahren nach Anspruch 10, dadurch gekennzeichnet, daß das Molverhältnis von gelöstem $SO_2$ zu Vanadium im Bereich von 1: 1 bis 3: 1 liegt.

**Revendications**

1 - Procédé pour la préparation d'acide adipique, dans lequel:

(1) du cyclohexanol et/ou de la cyclohexanone sont oxydés par l'acide nitrique en présence d'un catalyseur au vanadium, pour obtenir un produit réactionnel liquide comprenant de l'acide adipique,

(2) ce produit réactionnel est soumis à une cristallisation pour isoler l'acide adipique d'une liqueur-mère comprenant de l'acide nitrique, de l'acide succinique, de l'acide glutarique, de l'acide adipique résiduel, du vanadium, et

(3) le vanadium est récupéré à partir de la liqueurmère par échange de cations, caractérisé par l'amélioration consistant à introduire une quantité efficace d'anhydride sulfureux dans la liqueurmère avant la récupération par échange de cations, de façon à augmenter l'échange de cations.

2 - Procédé selon la revendication 1, dans lequel du cuivre est aussi introduit comme catalyseur et récupéré par l'échange de cations.

3 - Procédé selon l'une quelconque des revendications 1 ou 2, dans lequel le catalyseur au vanadium est un membre du groupe constitué du vanadium métallique, d'un acide du vanadium, d'un oxyde du vanadium et d'un sel du vanadium.

4 - Procédé selon la revendication 3, dans lequel le catalyseur au vanadium est $V_2O_5$.

5 - Procédé selon l'une quelconque des revendications 1 à 4, dans lequel au cours de l'échange d'ions, le $SO_2$ est formé in situ dans la liqueur-mère par addition d'un membre du groupe constitué des sulfites métalliques et des bisulfites métalliques.

6 - Procédé selon l'une quelconque des revendications 1 à 5, dans lequel au cours de l'échange d'ions, le $SO_2$ est présent dans un rapport molaire au vanadium de 1 : 1 à 5 : 1.

7 - Procédé selon la revendication 6, dans lequel le rapport molaire est de 1 : 1 à 3 : 1.

8 - Procédé selon l'une quelconque des revendications 1 à 7, dans lequel au moins une partie du procédé, y compris l'échange d'ions, est dans un système fermé et du $SO_2$ est introduit dans cette partie par barbotage.

9 - Procédé selon l'une quelconque des revendications 1 à 7, dans lequel le procédé est effectué dans un système ouvert et le $SO_2$ est injecté avec une installation de lavage à l'eau classique.

10 - Procédé selon l'une quelconque des revendications 1 à 5, dans lequel du $SO_2$ est ajouté à une partie de l'alimentation de l'échange d'ions dans une quantité telle que l'on obtienne une solution saturée, laquelle est à son tour ajoutée à l'alimentation d'échange d'ions de façon à réaliser un rapport molaire du $SO_2$ dissous au vanadium de 1 : 1 à 5 : 1.

11 - Procédé selon la revendication 10, dans lequel le rapport molaire du $SO_2$ dissous au vanadium est de 1 : 1 à 3 : 1.